# DEMANDE DE BREVET EUROPEEN

(11) **EP 4 035 653 A1**
(43) Date de publication de la demande: **03.08.2022**
(21) Numéro de dépôt: 21305117.0
(22) Date de dépôt: 29.01.2021
(51) Int. Cl.: A61K 8/9783, A61Q 19/08

(54) **EXTRAIT HUILEUX DE FLEURS DE GARDENIA JASMINOIDES ET COMPOSITIONS COSMÉTIQUES LE COMPRENANT**

(71) Demandeur: Chanel Parfums Beauté, 92200 Neuilly-sur-Seine (FR)
(72) Inventeur: RILHAC, Vincent, 93694 PANTIN CEDEX (FR); GILLET, MAEVA, 93694 PANTIN CEDEX (FR)
(74) Mandataire: Plasseraud IP

(57) **Abrégé**

L'invention concerne un extrait huileux de fleurs de *Gardénia jasminoides* obtenu par extraction au CO₂ supercritique de poudre de fleurs de *Gardénia jasminoides,* une composition cosmétique comprenant un tel extrait présentant notamment un effet anti-vieillissement pour la peau.

## Description

La présente invention a pour objet un extrait huileux de fleurs de *Gardenia jasminoides,* caractérisé en ce qu'il est susceptible d'être obtenu par extraction des fleurs au moyen de CO₂ supercritique, ainsi que son utilisation en cosmétique, pour la prévention et/ou le traitement d'altérations de la peau dus, notamment, au vieillissement ou à des mécanismes physiologiques liés au vieillissement ou à des troubles apparentés à ces mécanismes.

La peau est principalement constituée de trois couches, à savoir, en partant de la plus superficielle, l'épiderme, le derme et l'hypoderme.

La couche externe de la peau, l'épiderme, est stratifiée et contribue largement à assurer la protection de la peau vis-à-vis des agressions extérieures. Le derme est un tissu conjonctif assurant à la fois les fonctions de cohésion et de nutrition de la peau.

Le vieillissement cutané résulte de deux processus distincts et indépendants qui font intervenir des facteurs intrinsèques ou extrinsèques.

Le vieillissement intrinsèque ou chronobiologique correspond au vieillissement « normal » ou physiologique lié à l'âge.

Le vieillissement extrinsèque correspond au vieillissement provoqué d'une manière générale par l'environnement et plus particulièrement au photovieillissement dû à l'exposition au soleil.

La présente invention s'intéresse au vieillissement cutané intrinsèque ou physiologique ainsi qu'au vieillissement cutané extrinsèque.

Le vieillissement cutané est consécutif à une transformation des tissus conjonctifs et à l'abaissement de la capacité de régénération cellulaire. Cet effet se manifeste par l'apparition de ridules et de stigmates au cours du temps. La microcirculation diminue au niveau du derme superficiel. Les macromolécules telles que le collagène, l'élastine et les glycosaminoglycanes, dont l'acide hyaluronique est l'un des constituants, sont chimiquement modifiées. L'épaisseur même du derme régresse, les fibres sont dégradées et la peau perd ses propriétés biomécaniques et élastiques. Les phénomènes d'oxydation chimique et enzymatique augmentent avec l'âge et entraîne l'accroissement des réactions de pontage entre les fibres telles que les fibres de collagènes.

Les altérations associées au vieillissement peuvent se manifester de différentes manières, parmi lesquelles on peut citer :
- une désorganisation des fibres d'élastine entraînant une perte de fermeté, de souplesse et d'élasticité ou par l'apparition de télangiectasies;
- la perte d'éclat due à la réduction de la microcirculation et à un ralentissement du renouvellement cellulaire au niveau de l'épiderme et l'apparition de fines rides ou ridules ;
- le jaunissement de la peau qui développe un aspect parcheminé accompagné de l'apparition de taches pigmentaires associées à un dysfonctionnement de la synthèse de la mélanine (ou mélanogénèse) ;
- la sécheresse cutanée résultant d'une diminution de la fonction barrière de la couche cornée et à un ralentissement du renouvellement épidermique.

Il existe, de ce fait, un besoin de fournir un agent actif polyfonctionnel susceptible d'agir sur un ensemble de causes d'altérations de la peau dues au vieillissement et/ou à une modification des mécanismes physiologiques liés au vieillissement ou apparentés.

Les gardénias sont des plantes du genre *Gardenia,* un genre qui comprend environ 250 espèces de plantes à fleurs de la famille des Rubiacées, originaires des régions tropicales à subtropicales d'Afrique, d'Asie du Sud, d'Australasie et d'Océanie. Les extraits, généralement aqueux, de ses fleurs peuvent être recherchés pour être utilisés en parfumerie.

Toutefois, pour des formulations cosmétiques dans des compositions majoritairement non aqueuses, comme par exemple les compositions de maquillage, il est souhaitable de disposer d'extraits huileux de *Gardenia jasminoides.*

Si l'huile issue du fruit de *Gardenia jasminoides* a déjà été étudiée, peu de recherches scientifiques ont été réalisées sur les extraits de fleurs.

La demande CN104905999 a proposé un extrait huileux de fleurs de Gardénia, pour son utilisation dans des parfums. L'extrait décrit dans cette demande est obtenue par extraction des fleurs fraiches de Gardénia avec du CO₂ supercritique dans des conditions douces (35°C/15-20MPa) afin de préserver les molécules odorantes dans l'extrait.

Les auteurs de la présente invention ont maintenant mis en évidence, de façon tout à fait surprenante, qu'une huile extraite à partir des fleurs séchées de *Gardenia jasminoides* avec du CO₂ supercritique dans des conditions de température et de pression plus poussées, présentait une activité sur les symptômes dus au vieillissement, ou à des mécanismes physiologiques liés au vieillissement, ou à des troubles apparentés à ces mécanismes au niveau de l'épiderme et/ou du derme.

Cette constatation a conduit à la mise au point de nouvelles compositions cosmétiques non thérapeutiques, plus particulièrement utiles pour toutes les applications dans lesquelles on cherche à agir sur les symptômes dus au vieillissement, ou sur les mécanismes physiologiques liés au vieillissement, ou sur les troubles apparentés à ces mécanismes au niveau de l'épiderme et/ou du derme.

L'invention concerne donc, selon un premier aspect, un procédé de préparation d'un extrait huileux de fleurs de *Gardenia jasminoides,* comprenant au moins l'extraction au CO₂ supercritique de poudre de fleurs de *Gardenia jasminoides,* à une température comprise entre 40 et 80°C, de préférence entre 50 et 70°C et à une pression comprise entre 25 et 50 MPa, de préférence entre 30 et 45 MPa.

L'invention a également pour objet un extrait huileux de fleurs de *Gardenia jasminoides* obtenu à partir d'un tel procédé. L'extrait huileux de fleurs de *Gardenia jasminoides* selon l'invention enrichi molécules apolaires tels que des stérols , dans un solvant huileux.

L'invention concerne, selon un troisième aspect, une composition cosmétique comprenant, dans un milieu physiologiquement acceptable, au moins un extrait huileux de fleur de *Gardenia jasminoides* selon l'invention.

Enfin, l'invention a pour objet, selon un quatrième aspect, l'utilisation cosmétique non thérapeutique d'un extrait huileux de fleur de *Gardenia jasminoides* tel que décrit précédemment, pour la prévention et/ou le traitement d'altérations de la peau dus au vieillissement ou au photo-vieillissement.

### Brève description des figures

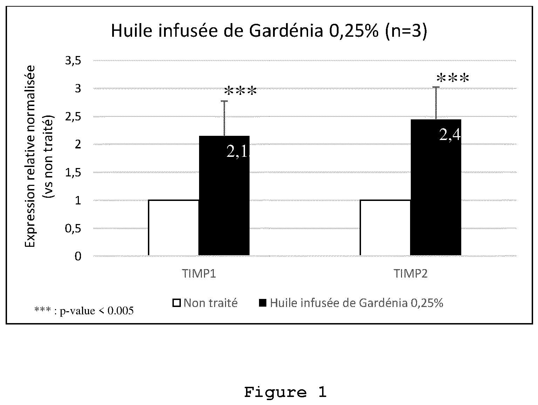

La **Figure 1** illustre l'augmentation de l'expression des gènes TIMP1 et TIMP2 dans des mélanocytes après traitement avec extrait huileux de fleur de *Gardenia jasminoides* selon l'invention à 0,25%

### Gardénia jasminoides

Le *Gardenia jasminoides J. Ellis* (synonyme *Gardenia florida*) est un genre de plantes à fleurs de la famille des Rubiacées.

Arbuste à feuilles persistantes, de la famille des Rubiacées, comme le Café et le Tiaré, atteignant 0,3 à 3 m de haut.

Les feuilles sont épaisses, vert foncé et brillantes.

Le genre Gardenia qui comprend environ 250 espèces de plantes à fleurs originaires des régions tropicales à subtropicales d'Afrique, d'Asie du Sud, d'Australasie et d'Océanie. Le *Gardenia jasminoides* est aussi connu sous les noms *Gardenia angustifolia Lodd, Gardenia grandiflora Lour, Genipa Florida (L.) Baill, Genipa grandiflora (Lour.) Baill, Jasminum capense Mill.*

La floraison spectaculaire offre des fleurs extrêmement parfumées et souvent doubles avec de très grandes pétales blanches, tout au long de l'année dans les climats chauds, de la fin du printemps au début de l'été dans les climats plus frais (mars-juillet).

Les fleurs blanches suivant les variétés présentent une odeur analogue à celui du jasmin. Le *Gardenia jasminoides* est, de fait, aussi connue sous le nom de Jasmin du cap. Le *Gardenia jasminoides* est utilisé dans la médecine traditionnelle chinoise pour diverses propriétés (émollientes, émétiques, diurétiques, vermifuges, antispasmodiques, antiseptiques, analgésiques). Le fruit est une baie jaune ou jaune orangé, ovoïde, contenant des graines faiblement coudées.

L'extrait selon l'invention est obtenu à partir des fleurs de *Gardenia jasminoides,* et de préférence, à partir des fleurs de couleur blanche, notamment des variétés "Kleim's Hardy" avec des fleurs simples de 5cm et "Crown jewel" avec une fleur double de 7 cm. De préférence, les fleurs de *Gardenia jasminoides* utilisées dans l'invention sont cultivées à Gaujacq en France.

L'extrait de fleurs de *Gardenia jasminoides* se présente de préférence sous forme de poudre séchée dispersible. Par dispersible, on entend que la poudre de fleurs de *Gardenia jasminoides* se présente sous une forme dissociée apte à être dispersée finement, et par exemple, la matière première est sous forme particulaire et de préférence pulvérulente. Les fleurs de *Gardenia jasminoides* fraiches sont, par exemple, dans un premier temps, isolées des tiges puis ouvertes et mises à plat sur des claies. Elles sont ensuite déshydratées en conditions douces, soit au soleil soit sous-vide à une température d'environ 40°C. Les fleurs sont de préférence séchées jusqu'à obtention d'une teneur en matière sèche supérieure à 80% et préférentiellement supérieure à 85%.

Les fleurs sont ensuite réduites en poudre dispersible par tout procédé de broyage classiquement connu de l'homme du métier, par exemple à température ambiante dans un broyeur à couteaux ou, selon un mode de réalisation préféré, par broyage à basse température. Pour un broyage à basse température, les fleurs sont de préférence refroidies à -80°C et immédiatement broyées dans un mixer à hélices à une température comprise entre -20 et -80°C, pour obtenir une poudre fine et régulière. La cryogénisation permet avantageusement d'assurer une meilleure conservation des propriétés hydratantes des molécules contenues dans les fleurs.

De préférence, la poudre dispersible de fleurs de *Gardenia jasminoides* mise en œuvre pour la préparation de l'extrait selon l'invention présente une taille moyenne de particules inférieure à 500 µm, préférentiellement inférieure à 300 µm. La poudre de fleurs de *Gardenia jasminoides* présente une odeur florale douce et une couleur allant de blanc crème à rouge brun.

### Procédé de préparation d'un extrait huileux de fleurs de Gardénia jasminoides

Selon une caractéristique essentielle de l'invention, l'extrait huileux de fleurs de *Gardenia jasminoides* , comprend au moins une étape d'extraction au CO₂ supercritique de poudre de fleurs de *Gardenia jasminoides,* dans des conditions de température et de pression spécifiques.

En effet, il est du mérite de la demanderesse d'avoir mis en évidence qu'en procédant à l'extraction huileuse des fleurs de *Gardenia jasminoides* au moyen de CO₂ supercritique dans des conditions de température et de pression particulières, il était possible d'obtenir un extrait enrichi en molécules apolaires tels que des stérols, lequel extrait a montré une efficacité tout à fait inattendue pour lutter contre le vieillissement et le photo-vieillissement cutané.

En particulier, l'extraction au CO₂ supercritique est opérée à une température comprise entre 40 et 80°C, de préférence entre 50 et 70°C et à une pression comprise entre 25 et 50 MPa, de préférence entre 30 et 45 MPa.

Selon un mode préféré de réalisation, le débit de CO₂ est d'au moins 150g/min, de préférence 200g/min.

Le CO₂ présente l'avantage d'être non-inflammable, non-toxique, inodore et facilement disponible puisque majoritairement présent dans l'air.

L'extraction au CO₂ supercritique est opérée en présence d'un solvant huileux, de préférence choisi parmi le squalane, le palmitate d'éthyl-2-hexyle, les triglycérides d'acide caprylique et caprique, et les huiles végétales.

Les une huile végétales peuvent par exemple être choisies parmi l'huile de camélia, l'huile de colza, l'huile de tournesol, l'huile d'olive, l'huile de sésame, l'huile de noyaux d'abricot, l'huile de pépin de raisin, l'huile d'amande douce, l'huile de carthame, l'huile de noisette, l'huile d'argan, l'huile de rosier muscat, l'huile d'onagre, l'huile de bourrache, la cire liquide de jojoba, et leurs mélanges.

Selon un mode préféré de réalisation, l'extraction au CO₂ supercritique est opérée en présence de squalane.

Il a en effet été observé que l'ajout de squalane durant l'extraction au CO₂ supercritique permettait une meilleure solubilisation des molécules d'intérêt présentes dans les fleurs de Gardénia. En outre, le squalane a l'avantage de présenter des propriétés biomimétiques avec la peau, puisqu'il est un composant naturel du sébum humain et forme sur la peau un film hydrolipidique. L'utilisation de squalane est compatible avec la mise en formulation de l'extrait dans une composition cosmétique : il confère à la peau un effet soyeux, et pénètre instantanément.

Selon un mode particulier de réalisation, le solvant huileux, de préférence le squalane, est introduit au cours de l'étape d'extraction au CO₂ supercritique en un ratio volumique CO2 : solvant huileux compris entre 200 : 1 et 50 : 1.

Lorsqu'on extrait environ 1kg de fleurs de *Gardenia jasminoides* par batch, l'étape d'extraction au CO₂ supercritique est de préférence conduite pendant une durée allant de 30 min à 6h, de préférence de 1h à 3h.

Dans le cadre de l'invention, à l'issue de l'étape d'extraction au CO₂ supercritique, la pression peut être abaissée pour passer le CO₂ à l'état gazeux, en particulier à une pression inférieure ou égale à 7.4 MPa, de préférence inférieure à 6 MPa pour garantir que tout le CO₂ est bien revenu à l'état gazeux.

Selon un mode particulier de réalisation, le procédé selon l'invention comprend au moins les étapes suivantes :
i. le séchage et le broyage de fleurs fraiches de *Gardenia jasminoides,*
ii. l'extraction au CO₂ supercritique de poudre de fleurs de *Gardenia jasminoides,* à une température comprise entre 40 et 80°C, de préférence entre 50 et 70°C et à une pression comprise entre 25 et 50 MPa, de préférence entre 30 et 45 MPa, pendant par exemple 30min à 6 heures, de préférence en présence d'un solvant huileux tel que le squalane,
iii. l'évaporation du CO₂ par abaissement de la pression jusqu'à 7.4 MPa ou moins, de préférence 6 MPa ou moins,
iv. optionnellement l'introduction d'un flux d'alcool, de préférence d'éthanol, pour collecter le mélange de poudre de fleurs de *Gardenia jasminoides* et de solvant, notamment de squalane, puis évaporation dudit alcool,
v. l'ajout d'un solvant huileux, notamment de squalane, jusqu'à un rapport pondéral fleur séchée:solvant huileux de 1:5 à 1 :20,
vi. la clarification de l'extrait huileux de fleurs de *Gardenia jasminoides.*

A titre de finition, le procédé selon l'invention comprend une ou plusieurs étape(s) de clarification de l'extrait huileux.

On entend par clarification toutes les séparations mécaniques connues de l'homme du métier. Elles peuvent par exemple être choisies parmi la filtration, la décantation, la centrifugation, l'essorage, ou une association de ces techniques.

Selon un mode préféré de réalisation, la clarification est opérée par filtration, sur une membrane de porosité inférieure ou égale à 4µm, voire de porosité 2 µm.

Les étapes de clarification permettent l'obtention d'un produit à la fois substantiellement limpide à l'œil et exempt de microparticules en suspension.

Selon un mode particulièrement préféré de réalisation, le procédé de préparation de l'extrait huileux de fleurs de *Gardenia jasminoides* selon l'invention, comprend les étapes suivantes :
i.1. le séchage des fleurs de *Gardenia jasminoides* au soleil ou à 40°C sous-vide,
i. 2. Le broyage des fleurs séchées et leur introduction dans le panier de l'extracteur CO₂ supercritique
ii.1. la compression du dioxyde de carbone à une pression équivalente à 45 MPa à l'échelle laboratoire ou 30MPa à l'échelle industrielle,
ii.2. le mélange du CO₂ compressé avec un solvant d'extraction cosmétique, en particulier le squalane, selon un rapport volumique CO₂ : squalane de 99 :1
ii.3. l'envoi du mélange obtenu à l'étape i.2. dans un échangeur thermique pour atteindre la température de 60°C
ii.4. l'extraction durant au moins 1h30, au cours de laquelle le mélange de l'étape i.2. passe à travers le panier contenant les fleurs séchées de Gardénia à un débit d'au moins 200g/min de sorte que les molécules extraites diffusent dans le mélange,
iii. la dépressurisation du mélange CO₂ supercritique/squalane/extrait de Gardénia pour atteindre environ 50 bars de sorte que le CO₂ redevienne gazeux,
iv.1. l'envoi d'un flux d'éthanol pour pousser le mélange squalane/extrait de Gardénia et envoyer le mélange dans les collecteurs du système d'extraction
iv.2. le soutirage du mélange grâce à la différence de pression entre le système et l'environnement externe, de manière à obtenir un mélange éthanol/squalane/extrait de Gardénia (le CO₂ étant évaporé)
iv.3. l'évaporation de l'éthanol résiduel est évaporé sous-vide,
v. L'ajout d'un solvant huileux, notamment de squalane, jusqu'à un rapport pondéral fleur séchée:solvant huileux de 1 :10, et
vi. la clarification de l'extrait huileux de fleurs de *Gardenia jasminoides* par filtration sur membrane de porosité inférieure à 1µm pour éliminer les précipités éventuels.

### Extrait huileux de fleurs de Gardénia jasminoides

L'invention objet de la présente demande couvre également un extrait huileux de fleur de *Gardenia jasminoides* obtenu au moyen du procédé précédemment décrit.

L'invention a aussi pour objet un extrait de fleur de *Gardenia jasminoides* huileux enrichi en molécules apolaires tels que des stérols.

### Composition cosmétique

La présente invention a encore pour objet une composition cosmétique comprenant, dans un milieu physiologiquement acceptable, au moins un extrait huileux de fleur de *Gardenia jasminoides*

La composition mise en œuvre selon l'invention comprend généralement, outre l'extrait décrit précédemment, un milieu physiologiquement acceptable et de préférence cosmétiquement acceptable, c'est-à-dire qui convient à une utilisation en contact avec la peau humaine sans risque de toxicité, d'incompatibilité, d'instabilité, de réponse allergique et notamment qui ne provoque pas de sensations d'inconfort (rougeurs, tiraillements, picotements.

Avantageusement, ladite composition cosmétique ou dermatologique peut se présenter sous la forme d'une poudre, d'une émulsion, d'une microémulsion, d'une nanoémulsion, d'une suspension, d'une solution d'une lotion, d'une crème, d'un gel aqueux ou hydroalcoolique, d'une mousse, d'un sérum, d'une solution ou d'une dispersion pour aérosol, ou d'une dispersion de vésicules lipidiques.

Dans le cas d'une émulsion, il peut s'agir d'une émulsion eau-dans-huile ou huile-dans-eau.

La composition cosmétique ou dermatologique selon l'invention peut comprendre également un solvant choisi en fonction des différents ingrédients et de la forme d'administration.

A titre d'exemples, on peut citer l'eau (de préférence de l'eau déminéralisée ou des eaux florales), un alcool tel que l'éthanol.

Ladite composition cosmétique peut également comprendre, en outre de l'extrait selon l'invention :
- au moins un additif usuel dans le domaine, tel que par exemple au moins un composé choisi parmi un agent émollient ou humectant, un agent gélifiant et/ou épaississant, un agent tensioactif, une huile, un agent actif, un colorant, un conservateur, un agent antioxydant, un agent actif, une poudre organique ou inorganique, un filtre solaire et un parfum.
- un ou plusieurs agent(s) **humectant(s),** tels que les polyols (glycérine, diglycérine, le propylène glycol, le caprylyl glycol, le pentylène glycol, l'hexanediol), les sucres, les glycosaminoglycanes tels que l'acide hyaluronique et ses sels et esters; et les polyquaterniums tel que le lipidure PMB. Ledit agent humectant sera présent dans la composition à une teneur de l'ordre de 0 à 30%, de préférence 0.005 à 10% en poids total de la composition.
- Un ou plusieurs agent(s) **émollient(s)** qui peuvent être choisi(s) par exemple parmi les esters tels que les esters de jojoba, les esters d'acides gras et d'alcool gras (octyldodecyl myristate, triethylhexanoin, Dicaprylyl carbonate, Isostearyl isostearate, caprylic/capric triglyceride), les beurres tels que les beurres de karité (butyrospernum parkii butter extract, shea butter ethyl esters, commercialisés sous les noms de LIPEX SHEASOFT, LIPEX SHEA-U, LIPEX SHEA, LIPEX SHEALIGHT, LIPEX SHEA TRIS) ou de moringa (moringa oil/hydrogenated moringa oil esters), les cires (acacia decurrens flower wax & helianthus annuus cera seed seed wax, C10-18 triglycerides), les huiles végétales, le phytosqualane, les alcanes (undecane, tridecane). Ledit agent émollient sera présent dans la composition à une teneur de l'ordre de 0,1 à 30%, de préférence 0,5 à 10% en poids total de la composition.
- Un ou plusieurs agent(s) **gélifiants**(s) et/ou épaississant(s) de la phase aqueuse, choisi par exemple parmi les dérivés cellulosiques, gommes d'origine végétale (guar, caroube, alginates, carraghenanes, pectines), d'origine microbienne (xanthane), les argiles (laponite), les homo- et copolymères réticulés ou non, hydrophiles ou amphiphiles, d'acide acryloylméthylpropane sulfonique (AMPS) et/ou d'acrylamide et/ou d'acide acrylique et/ou de sels ou d'esters d'acide acrylique (commercialisés sous les noms ARISTOFLEX AVC, Aristoflex AVS, Aristoflex HMB, SIMULGEL NS, Simulgel EG, Simulgel 600, Simulgel 800, Pemulen, carbopol, Sepiplus 400, Seppimax zen, Sepiplus S, COSMEDIA SP). Ledit agent gélifiant et/ou épaississant sera présent dans la composition à une teneur de l'ordre de 0,1 à 10% en poids total de la composition.
- Un ou plusieurs agent(s) **tensioactif**(s), tels que les lecithines, les dérivés de polyglycérol, les dérivés de sucre (les dérivés de glucosides ou de xylosides commercialisés sous le nom MONTANOV 68, MONTANOV 202, Montanov 82, MONTANOV L, EASYNOV), les phosphates (C20-22 alkyl phosphate commercialisé sous le nom SENSANOV WR). Ledit agent tensioactif, sera présent dans une teneur de l'ordre de 0,1 à 8%, de préférence 0,5 à 3% en poids, par rapport au poids total de la composition.
- Un ou plusieurs agent(s) **actif**(s) d'origine naturelle, biotechnologique ou synthétique ayant une activité biologique et ayant une efficacité sur la peau via des sites biologiques, par exemple choisi parmi les vitamines tels que la vitamine C et ses dérivés (ascorbyl glucoside, 3-o-ethyl ascorbic acid, le tétraisopalmitate d'ascorbyle) ,la vitamine A et ses dérivés, la vitamine E et ses dérivés, la vitamine B3 ou Niacinamide, le panthénol, les oligo éléments, l'allantoïne, l'adenosine, les peptides (Palmitoyl tetrapeptide-7, Palmitoyl Tripeptide-1, Palmitoyl Pentapeptide-4, Acetyl Dipeptide-1 Cetyl Ester, Acetyl Tetrapeptide-5 commercialisés sous le nom NP RIGIN, MATRIXYL 3000, IDEALIFT, EYESERYL), les extraits végétaux (glycyrrhiza glabra extract, centella asiatica leaf extract, secale cereale seed extract), les extraits de levures, les alpha hydroxyacides tels que l'acide glycolique ou lactique, l'acide tranexamique et ses dérivés tel que le cetyl tranexamique ester etc. Ledit agent actif sera présent dans la composition à une teneur de l'ordre de 0,1 à 10% en poids total de la composition.

D'autres additifs habituellement utilisés en cosmétique peuvent également être présents dans la composition selon l'invention, notamment des conservateurs, des agents antioxydants ou des parfums bien connus dans le domaine technique.

L'homme du métier est en mesure de choisir, parmi l'ensemble de ces éventuels additifs, aussi bien la nature que la quantité de ceux qui seront ajoutés à la composition, de telle sorte que celle-ci conserve l'ensemble de ses propriétés.

L'invention a également pour objet l'utilisation cosmétique de l'extrait huileux de fleur de *Gardenia jasminoides* pour prévenir et/ou traiter les altérations de la peau dues au vieillissement ou au photo-vieillissement.

En particulier, l'extrait huileux de fleur de *Gardenia jasminoides* peut être utilisé pour conférer un effet anti-âge lissant.

Dans ce mode de réalisation, l'extrait ou la composition est appliqué sur une peau altérée mais non pathologique.

L'invention a encore pour objet l'utilisation cosmétique non thérapeutique d'un extrait huileux de fleur de *Gardenia jasminoides* tel que décrit précédemment, en tant qu'un agent inhibiteur de l'activité des métalloprotéinases matricielles (MMPs).

Enfin, l'invention vise l'utilisation cosmétique non thérapeutique d'un extrait huileux de fleur de *Gardenia jasminoides* tel que décrit précédemment, en tant qu'un agent stimulateur du métabolisme cellulaire, en particulier stimulateur de l'expression des gènes TIMP 1 et/ou TIMP 2.

L'invention sera maintenant illustrée par les exemples non limitatifs suivants.

### Exemple : Activation de deux inhibiteurs des MMP dans des mélanocytes humains normaux traités avec l'extrait huileux de de Gardénia jasminoides

### Préparation de l'extrait :

Un extrait huileux de *Gardenia jasminoides* selon l'invention a été préparé, selon les étapes suivantes :
a) Les fleurs fraichement récoltées sont séchées au soleil ou à 40°C sous-vide pour préserver leur qualité puis broyées finement
b) Les fleurs broyées sont disposées dans le panier de l'extracteur CO₂ supercritique
c) Le dioxyde de carbone (CO₂) est compressé à une pression équivalente à 450 bars à l'échelle laboratoire ou 300 bars à l'échelle industrielle
d) Le CO₂ compressé est ensuite mélangé avec un solvant cosmétique, squalane, selon un rapport 99/1 (CO₂ : squalane, v/v)
e) Le mélange est envoyé dans un échangeur thermique pour atteindre la température de 60°C
f) Durant au moins 1h30, le mélange passe à travers le panier contenant les fleurs séchées de gardénia à un débit d'au moins 200g/min, les molécules extraites diffusent dans le mélange
g) Le mélange CO₂ supercritique/squalane/extrait de Gardénia est ensuite dépressurisé pour redescendre à environ 50 bars, le CO₂ redevient gazeux
h) Un flux d'éthanol est envoyé à ce niveau pour pousser le mélange squalane/extrait de Gardénia et envoyer le mélange dans les collecteurs du système d'extraction
i) Le mélange est soutiré grâce à la différence de pression entre le système et l'environnement externe, on obtient un mélange éthanol/squalane/extrait de Gardénia (le CO₂ étant évaporé)
j) L'éthanol résiduel est évaporé sous-vide
k) On rajoute autant de squalane que nécessaire pour que la masse finale de l'extrait soit 10 fois supérieure à la masse de plante mise en jeu (exemple : pour 100g de fleurs séchées mise en œuvre, ajouter 1000g de squalane) .
l) L'extrait est filtré sur membrane à 1µm pour éliminer les précipités éventuels.

### Protocole :

Des mélanocytes épidermiques humains normaux issus de 3 donneurs différents ont été cultivés en plaque 6 puits en milieu M254 complété avec 1% de HMGS pendant 72h à 37°C et 5% de CO₂. A 70% de confluence, les cellules ont été incubées ou non (condition non traitée) pendant 24h avec une concentration non cytotoxique (0.25%) extrait huileux de *Gardenia jasminoides* préparé. Chaque condition a été effectuée en duplicat. Les ARN totaux ont été extraits à l'aide du kit RNeasy 96 Plate Extraction (Qiagen) en suivant les recommandations du fournisseur. La quantité et la qualité des ARN ont été évaluées à l'aide d'un spectrophotomètre (Multiskan GO, Thermo Fisher). Les ADN complémentaires ont ensuite été synthétisés à partir d'un microgramme d'ARN selon la procédure décrite par le fournisseur (iScript SUPER mix, Biorad) et utilisés pour déterminer le niveau d'expression de gènes d'intérêt par PCR quantitative. L'analyse a été effectuée par la méthode de comparaison des Ct après normalisation par rapport à l'expression de gènes de référence, en utilisant le logiciel Biorad Maestro CFX.

### Résultats :

Les résultats obtenus après traitement des mélanocytes par extrait huileux de *Gardenia jasminoides* selon l'invention HG) sont présentés dans la Figure 1. Une augmentation significative de l'expression des gènes TIMP 1 et 2 (Tissue inhibitor of metalloproteinase) a été observée dans des mélanocytes traités avec 0.25% de HG par rapport à des mélanocytes non traités. Les mélanocytes traités ont ainsi exprimé en moyenne (n=3) 2,15 fois plus de TIMP1 et 2,45 fois plus de TIMP2 que les mélanocytes non traités.

| | | **Donneur 1** | | | **Donneur2** | | | **Donneur3** | | |
|---|---|---|---|---|---|---|---|---|---|---|
| **Cible** | **Condition** | Ct moyen | Expression relative normalisée | P-Value | Ct moyen | Expression relative normalisée | P-Value | Ct moyen | Expression relative normalisée | P-Value |
| **TIMP1** | **HG 0.25%** | 22,80 | 2,07887 | 0,001588 | 25,88 | 1,56516 | 0,002353 | 22,01 | 2,80538 | 0,001649 |
| | **Non traitée** | 25,68 | 1,00000 | | 27,89 | 1,00000 | | 25,98 | 1,00000 | |
| **TIMP2** | **HG 0.25%** | 22,36 | 2,82179 | 0,001940 | 23,69 | 1,78326 | 0,005172 | 19,89 | 2,74187 | 0,003784 |
| | **Non traitée** | 25,69 | 1,00000 | | 25,88 | 1,00000 | | 23,82 | 1,00000 | |

Il existe 4 membres de la famille des TIMPs (TIMP1 à 4), agissant comme inhibiteurs spécifiques de l'activité des métalloprotéinases matricielles (MMPs). Les MMPs sont connues pour être impliquées dans la dégradation de la matrice extracellulaire du derme et le vieillissement de la peau. Notamment, à la suite d'une exposition aux rayons UV, une forte production de MMPs est observée, ce qui contribue au photo-vieillissement cutané. Or, il a été montré que les mélanocytes ont une activité protéolytique très élevée, en secrétant notamment des MMPs. Ainsi, l'extrait huileux de fleur de *Gardenia jasminoides* selon l'invention pourrait inhiber l'activité des MMPs en stimulant l'expression des TIMP dans les mélanocytes et donc participer à prévenir et/ou traiter le vieillissement et/ou le photo-vieillissement par un effet anti-âge lissant par exemple.

### EXEMPLE : Composition cosmétique

Les compositions suivantes peuvent être préparées de façon classique pour l'homme du métier. Les quantités indiquées ci-dessous sont exprimées en pourcentages pondéraux. Les ingrédients en majuscules sont identifiés conformément à la dénomination INCI.

### A - émulsion huile/eau

| **Nom INCI** | **(% W/W)** |
|---|---|
| LIMNANTHES ALBA (MEADOWFOAM) SEED OIL | 1-10 |
| BUTYROSPERMUM PARKII BUTTER (LIPEX SHEASOFT) | 1-10 |
| BUTYROSPERNUM PARKII BUTTER EXTRACT (LIPEX SHEA TRIS) | 1-10 |
| CAMELLIA OLEIFERA SEED OIL | 1-10 |
| CETYL ETHYLHEXANOATE | 1-5 |
| SQUALANE | |
| SODIUM ACRYLATES COPOLYMER & LECITHIN | 0.1-5 |
| ACRYLATES/C10-30 ALKYL ACRYLATE CROSSPOLYMER | 0.1-2 |
| GLYCERYL STEARATE & PEG-100 STEARATE | 0.1-5 |
| XANTHANE GUM | 0.01-5 |
| HYDROXYETHYL CELLULOSE | 1-10 |
| SILICA | 0.1-10 |
| SODIUM HYALURONATE | 0.01-3 |
| GLYCERIN | 1-30 |
| POLYQUATERNIUM-51 | 1-10 |
| ADENOSINE | 0.1-0.5 |
| NIACINAMIDE | 0.1-5 |
| PALMITOYL TETRAPEPTIDE-7 | 1-5 |
| TRANEXAMIC CETYL ESTER | 0.001-5 |
| ALLANTOINE | 0.001-5 |
| TOCOPHERYL ACETATE | 0.1-5 |
| EXTRAIT SELON L'INVENTION | 0.001-10 |
| YEAST EXTRACT | 0.1-5 |
| GLYCYRRHIZA GLABRA EXTRACT | 0.1-5 |
| ASCORBYL GLUCOSIDE | 0.001-5 |
| GLYCOLS (CAPRYLYL GLYCOL AND/OR PENTYLENE GLYCOL AND/OR BUTYLENE GLYCOL AND/OR PROPANEDIOL) | 0,1-10 |
| WATER | Qs 100 |

### b - émulsion huile/eau crème

| **Nom INCI** | **(% w/w)** |
|---|---|
| BEHENYL ALCOHOL | 1-5 |
| CETYL ALCOHOL | 0.1-5 |
| LAUROYL LYSINE | 1-5 |
| CAMELLIA OLEIFERA SEED OIL | 1-10 |
| CETYL ETHYLHEXANOATE | 1-5 |
| SQUALANE | 1-10 |
| HYDROGENATED LECITHIN & GLYCINE SOJA (SOYBEAN) STEROLS | 1-5 |
| POLYGLYCERYL-6 POLYHYDROXYSTEARATE (AND) POLYGLYCERYL-6 POLYRICINOLEATE | 1-7 |
| XANTHAN GUM | 0, 01-2 |
| AGAR | 0.1-5 |
| ADENOSINE | 0.1-0.5 |
| NIACINAMIDE | 0.1-5 |
| SECALE CEREALE (RYE) SEED EXTRACT | 0.1-5 |
| PALMITOYL TETRAPEPTIDE-7 | 1-5 |
| TRANEXAMIC CETYL ESTER | 0.001-5 |
| ASCORBYL GLUCOSIDE | 0.001-5 |
| YEAST EXTRACT | 1-3 |
| SACCHARIDE ISOMERATE | 1-5 |
| EXTRAIT SELON L'INVENTION | 0.001-10 |
| GLYCYRRHIZA GLABRA EXTRACT | 0.001-5 |
| WATER | Qs 100 |

Ces compositions peuvent être appliquées tous les jours, matin et/ou soir, sur la peau.

## Revendications

1. Procédé de préparation d'un extrait huileux de fleurs de *Gardenia jasminoides,* comprenant au moins l'extraction au CO₂ supercritique de poudre de fleurs de *Gardenia jasminoides,* à une température comprise entre 40 et 80°C, de préférence entre 50 et 70°C et à une pression comprise entre 25 et 50 MPa, de préférence entre 30 et 45 MPa.

2. Procédé selon la revendication 1, **caractérisé en ce que** l'extraction au CO₂ supercritique est opérée en présence d'un solvant huileux, de préférence choisi parmi le squalane, le palmitate d'éthyl-2-hexyle, les triglycérides d'acide caprylique et caprique, et les huiles végétales, plus préférentiellement le squalane.

3. Procédé selon la revendication 2, **caractérisé en ce que** le solvant huileux, de préférence le squalane est introduit en un ratio volumique CO₂ : solvant huileux compris entre 200 : 1 et 50 : 1.

4. Procédé selon l'une quelconque des revendications précédentes, **caractérisé en ce que** la poudre de fleurs de *Gardenia jasminoides* est obtenue par séchage puis broyage des fleurs fraiches de *Gardenia jasminoides.*

5. Procédé selon l'une quelconque des revendications précédentes, **caractérisé en ce qu'**à l'issue de l'étape d'extraction au CO₂ supercritique, la pression est abaissée pour passer le CO₂ à l'état gazeux, en particulier à une pression inférieure ou égale à 7.4 MPa, de préférence inférieure ou égale à 6 MPa.

6. Procédé selon l'une quelconque des revendications précédentes, **caractérisé en ce qu'**il comprend au moins les étapes suivantes :
i. le séchage et le broyage de fleurs fraiches de *Gardenia jasminoides,*
ii. l'extraction au CO₂ supercritique de poudre de fleurs de *Gardenia jasminoides,* à une température comprise entre 40 et 80°C, de préférence entre 50 et 70°C et à une pression comprise entre 25 et 50 MPa, de préférence entre 30 et 45 MPa, par exemple pendant 30min à 6 heures, de préférence en présence d'un solvant huileux tel que le squalane,
iii. l'évaporation du CO₂ par abaissement de la pression jusqu'à 7,4 MPa ou moins, de préférence 6 MPa ou moins,
iv. optionnellement l'introduction d'un flux d'alcool, de préférence d'éthanol, pour collecter le mélange de poudre de fleurs de *Gardenia jasminoides* et de solvant, notamment de squalane, puis évaporation dudit alcool,
v. l'ajout d'un solvant huileux, notamment de squalane, jusqu'à un rapport pondéral fleur séchée:solvant huileux de 1:5 à 1 :20,
vi. la clarification de l'extrait huileux de fleurs de *Gardenia jasminoides.*

7. Procédé selon la revendication 6, **caractérisé en ce que** l'étape vi. de clarification est opérée par filtration, sur une membrane de porosité inférieure à 1µm.

8. Extrait huileux de fleur de *Gardenia jasminoides,* **caractérisée en ce qu'**il est obtenu au moyen d'un procédé selon l'une quelconque des revendications 1 à 7.

9. Composition cosmétique comprenant, dans un milieu physiologiquement acceptable, au moins un extrait huileux de fleur de *Gardenia jasminoides* selon la revendication 8.

10. Utilisation cosmétique non thérapeutique d'un extrait huileux de fleur de *Gardenia jasminoides* selon la revendication 8, pour prévenir et/ou traiter les altérations de la peau dues au vieillissement ou au photo-vieillissement.
